(19) Europäisches Patentamt
European Patent Office
Office européen des brevets

(11) Publication number : 0 537 993 A1

(12) # EUROPEAN PATENT APPLICATION

(21) Application number : 92309339.7

(22) Date of filing : 14.10.92

(51) Int. Cl.[5] : C07D 211/70, A61K 31/445

(30) Priority : 17.10.91 US 778022

(43) Date of publication of application :
21.04.93 Bulletin 93/16

(84) Designated Contracting States :
AT BE CH DE DK ES FR GB GR IT LI LU NL PT SE

(71) Applicant : ELI LILLY AND COMPANY
Lilly Corporate Center
Indianapolis Indiana 46285 (US)

(72) Inventor : Ward, John Stanley
241 East Brunswick
Indianapolis, Indiana 46227 (US)

(74) Representative : Tapping, Kenneth George et al
Erl Wood Manor
Windlesham Surrey, GU20 6PH (GB)

(54) **Nicotinic activity of a series of arecolones and isoarecolones.**

(57) Series of 1,2,5,6-tetrahydro-3-pyridyl ketones and 1,2,3,6-tetrahydro-4-pyridyl ketones and the physiologically acceptable acid addition salts thereof have nicotinic agonist or antagonist activity and are useful in the treatment of Alzheimer's disease, Parkinson's disease and other central nervous system disorders, pain, gastrointestinal disorders, diabetes.

EP 0 537 993 A1

This invention relates to a series of arecolones and isoarecolones, the synthesis thereof, their selective affinity for nicotinic receptors and their use in treating Alzheimer's disease, other central nervous system disorders, pain, gastrointestinal disorders and diabetes.

Arecolone is widely known in the art as an important intermediate in the synthesis of cholinergic agonists and antagonists as well as other pharmaceuticals.

Isoarecolone is known as a nicotinic agonist and intermediate in the synthesis of other nicotinic agonists.

Reavill, C. disclosed that isoarecolone methiodide and isoarecolone HCl could inhibit binding and produce nicotine-like discriminative stimulus effects in rats. Reavill, C., et al., Neuropharmacology 26, 789-792 (1987).

Moreover, Spivak, C. found that isoarecolone methiodide (1-methyl-4-acetyl-1,2,3,6-tetrahydropyridine) was a very potent agonist which binds to nicotinic and muscarinic receptors. Spivak, C., et al., Mol. Pharmacol. 36, 177-184 (1989).

This invention provides a new synthesis for a series of arecolones and isoarecolones, their selective affinity for nicotinic receptors and their use in treating Alzheimer's disease and other central nervous system related disorders.

This invention relates to compounds of the formula

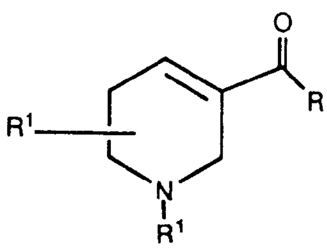

I

or

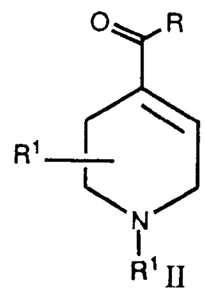

II

wherein

R is ($C_2$-$C_6$) alkyl, ($C_2$-$C_6$) alkenyl, ($C_2$-$C_6$) alkynyl, ($C_2$-$C_6$) alkoxyalkyl, ($C_2$-$C_6$) alkylthioalkyl, ($C_2$-$C_6$) alkylaminoalkyl, ($C_3$-$C_6$) cycloalkyl, ($C_1$-$C_6$) alkyl substituted with ($C_3$-$C_6$) cycloalkyl, or ($C_1$-$C_6$) polyfluoroalkyl;

each $R^1$ independently is hydrogen or ($C_1$-$C_3$) alkyl; or a physiologically acceptable acid addition salt thereof.

The invention also provides a pharmaceutical formulation comprising a compound of formula I or II in combination with a pharmaceutically acceptable excipient therefor.

Another embodiment of the invention is a method for treating a mammal with a central nervous system related disorder wherein said disorder is related to nicotinic agonist or antagonist activity, which comprises administering an effective amount of the compound or a pharmaceutically acceptable salt thereof.

The general chemical terms used in the formulae above have their usual meaning. For example, the term "alkyl" represents a straight or branched alkyl chain having the indicated number of carbons. "($C_1$-$C_3$) alkyl" groups are methyl, ethyl, n-propyl, and isopropyl.

The term "($C_2$-$C_6$) alkyl" includes both straight and branched chain alkyl and includes ethyl, n-propyl, isopropyl, n-butyl, sec-butyl, isobutyl, tert-butyl, n-pentyl, 2-methylbutyl, 3-methylbutyl, n-hexyl, 4-methylpentyl, and the like.

Cycloalkyl refers to a radical of a saturated cyclic hydrocarbon with the indicated number of carbons such as cyclopropyl, cyclobutyl, cyclopentyl, or cyclohexyl.

The term "($C_2$-$C_6$) alkenyl" refers to olefinically unsaturated hydrocarbons, such as $-CH=CH_2$, $-CH_2CH=CH_2$, $-CH_2CH_2CH=CH_2$, -CH(CH3)CH=$CH_2$, and the like.

Polyfluoro in "$C_1$-$C_6$ polyfluoroalkyl" means ($F_2$-$F_{13}$)-alkyl.

The term "($C_2$-$C_6$) alkynyl" refers to unsaturated hydrocarbons which contain triple bonds, such as $-C{\equiv}CCH_3$, $-CH_2C{\equiv}CH$, $-CH_2CH_2C{\equiv}CH$, $-CH(CH_3)C{\equiv}H$, and the like.

($C_2$-$C_6$) alkoxyalkyl means a group of 2 to 6 carbons which is interrupted by an 0. ($C_2$-$C_6$)alkylthioalkyl means a group of 2 to 6 carbons which is interrupted by a S. ($C_2$-$C_6$) alkylaminoalkyl means a group of 2 to 6 carbons which is interrupted by a N.

The term "physiologically acceptable acid addition salt", synonymous with and used interchangeably herein with "pharmaceutically acceptable salt", encompasses that salt that forms by acid-base reactions with basic groups (such as amino groups) and acidic groups, particularly carboxylic acid groups, on the compounds of formula I or II. Thus, a physiologically acceptable acid addition salt of the present invention can be prepared by conventional chemical methods from the compounds of formula I or II which contain a basic or acidic moiety. Generally, a salt is prepared by reacting the free base or acid with a stoichiometric amount or with an excess

of the desired salt-forming acid or base in a suitable solvent or combination of solvents. Suitable salt-forming acids include inorganic acids such as hydrochloric, hydrobromic, sulfuric, sulfamic, phosphoric, nitric, and the like; organic acids such as acetic, propionic, succinic, glycolic, stearic, lactic, citric, malic, tartaric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethanedisulfonic, oxalic, benzenesulfonic, picric, cinnamic, and like acids. Bases which find use for preparation of salts of compounds of formula I or II having an acidic moiety include alkali or alkaline earth metal hydroxides such as sodium, potassium, lithium, calcium, or magnesium hydroxides, ammonia, or organic bases such as benzylamine, dibenzylamine, dibenzylethylenediamine, triethylamine, trimethylamine, piperidine, pyrrolidine, 2-hydroxyethylamine, bis(2-hydroxyethyl) amine, phenylethyl-benzylamine, and like organic amines.

It has been speculated that nicotine acts in regions of the brain that are rich in binding sites, such as the mesolimbic dopamine system. Stolerman, I., New Scientist 128, 33-35 (1990). Biochemical studies support this theory by showing that nerve terminals of the mesolimbic dopamine system carry nicotinic receptors. Id. It has also been proposed that the neurotransmitter, 5-HT, may possibly act as $5\text{-}HT_3$ receptors to enhance the effect of nicotine on the dopamine system. Id. Radioligand binding studies using [$^3$H]-nicotine and [$^3$H]-acetylcholine have shown that Alzheimer's disease is associated with decreases in the cortical density of nicotinic acetylcholine receptor binding sites. Schroder, H. et al., Neurology of Aging 12, 259-262 (1991).

The compounds of this invention have cholinergic nicotinic activity. They displace the selective nicotinic ligand [$^3$H]-N-(methylcarbamyl)choline([$^3$H]MCC) from rat membranes. This affinity for nicotinic receptors is indicative of nicotinic agonist or antagonist activity; and compounds with such activity will find their use in the treatment of central nervous system disorders such as Alzheimer's disease, Parkinson's disease, neuroleptic disorders, tardive dyskinesia, psychosis or Gilles de la Tourette syndrome; other CNS disorders where nicotinic agonist or antagonist activity is believed to be a causative factor, such as panic attacks and other forms of anxiety; gastrointestinal disorders such as irritable bowel syndrome, ulcers, excess pancreatic or gastric secretion, acute pancreatitis, motility disorders, neoplasms of gastrointestinal origin; in modulating appetite regulatory systems; pain, and diabetes.

A group of representative compounds according to the invention will be mentioned by name, to assure that the reader of this document fully understands the compounds.

1-(1,2,5,6-tetrahydro-1-methyl-3-pyridinyl)-1-propanone hydrochloride,
1-(1,2,5,6-tetrahydro-3-pyridinyl)-1-butanone hydrochloride,
1-(1,2,5,6-tetrahydro-1-methyl-3-pyridinyl)-1-pentanone ethanedioate (1:1),
1-(1,2,5,6-tetrahydro-1-methyl-3-pyridinyl)-cis-2-buten-1-one maleate (1:1),
1-(1,2,5,6-tetrahydro-1-methyl-3-pyridinyl)-2-propyn-1-one fumarate (1:1),
(1,2,5,6-tetrahydro-1-ethyl-3-pyridinyl) cyclopropylmethanone hydrobromide,
1-(1,2,5,6-tetrahydro-1-ethyl-3-pyridinyl)-2-methoxyethanone citrate (1:1),
1-(1,2,5,6-tetrahydro-1-methyl-3-pyridinyl)-2-cyclobutylethanone hydrochloride,
1-(1,2,5,6-tetrahydro-1-methyl-3-pyridinyl)-3-methylthiopropan-1-one maleate (1:1),
1-(1,2,5,6-tetrahydro-1-methyl-3-pyridinyl)-1-hexanone hydrogensulfate (1:1),
1-(1,2,5,6-tetrahydro-1-propyl-3-pyridinyl)-4-pentyn-1-one hydrobromide,
1-(1,2,5,6-tetrahydro-1-methyl-3-pyridinyl)-trans-3-hexen-1-one citrate (1:1),
1-(1,2,5,6-tetrahydro-1-methyl-3-pyridinyl)-5-hexen-1-one ethanedioate (1:1),
1-(1,2,5,6-tetrahydro-1-methyl-3-pyridinyl)-2-dimethylaminoethanone hydrochloride,
1-(1,2,5,6-tetrahydro-1-methyl-3-pyridinyl)-2,2,2-trifluoroethanone fumarate (1:1),
1-(1,2,5,6-tetrahydro-1-methyl-3-pyridinyl)-2,2,3,3,3-pentafluoropropan-1-one ethanedioate (1:1),
1-(1-methyl-1,2,3,6-tetrahydro-4-pyridinyl)-1-propanone ethanedioate (1:1),
1-(1-methyl-1,2,3,6-tetrahydro-4-pyridinyl)-1-butanone ethanedioate (1:1),
1-(1-methyl-1,2,3,6-tetrahydro-4-pyridinyl)-1-pentanone hydrochloride,
1-(1-methyl-1,2,3,6-tetrahydro-4-pyridinyl)-2-butyn-1-one ethanedioate (1:1),
1-(1-methyl-1,2,3,6-tetrahydro-4-pyridinyl)-2-methyl-1-butanone ethanedioate (1:1),
1-(1-ethyl-1,2,3,6-tetrahydro-4-pyridinyl)-*cis*-3-penten-1-one hydrobromide,
1-(1-propyl-1,2,3,6-tetrahydro-4-pyridinyl)-1-propenone maleate (1:1),
1-(1,2-dimethyl-1,2,3,6-tetrahydro-4-pyridinyl)-ethanone fumarate (1:1),
1-(1,5-dimethyl-1,2,3,6-tetrahydro-4-pyridinyl)-1-butanone hydrogensulfate,
1-(1-methyl-1,2,3,6-tetrahydro-4-pyridinyl)-3-ethylthiopropan-1-one hydrochloride,
1-(1-methyl-1,2,3,6-tetrahydro-4-pyridinyl)-2,2-dimethylpropan-1-one maleate (1:1),
1-(1-methyl-5-ethyl-1,2,3,6-tetrahydro-4-pyridinyl)-ethanone ethanedioate (1:1),
1-(1-propyl-1,2,3,6-tetrahydro-4-pyridinyl)-3-methylpentan-1-one hydrochloride,
1-(1-methyl-1,2,3,6-tetrahydro-4-pyridinyl)-3-cyclopentylpropan-1-one hydrogenbromide,

(1-methyl-1,2,3,6-tetrahydro-4-pyridinyl)-cyclobutylmethanone fumarate (1:1),
1-(1-ethyl-1,2,3,6-tetrahydro-4-pyridinyl)-3-buten-1-one maleate (1:1), and
1-(6-methyl-1,2,3,6-tetrahydro-4-pyridinyl)-ethanone hydrochloride.

Certain classes of the compounds of this invention are preferred. The following paragraphs describe such preferred classes.

(a) the compound of formula I or II wherein R is ($C_2$-$C_6$) alkyl.
(b) the compound of formula I or II wherein R is ($C_2$-$C_6$) alkynyl.
(c) the compound of formula I or II wherein R is $-CH_2CH_3$.
(d) the compound of formula I or II wherein R is $-(CH_2)_2CH_3$.
(e) the compound of formula I or II wherein R is $-C\equiv CCH_3$.
(f) the compound of formula II wherein R is $-C(CH_3)_2CH_3$.
(g) the compound of formula I or II wherein R is alkyl, alkenyl, alkynyl.
(h) the compound of formula I or II wherein R is alkoxyalkyl, alkylthioalkyl, alkylaminoalkyl.
(i) the compound of formula I or II wherein $R^1$ is methyl.
(j) The compound is a salt.

It will be understood that the above classes may be combined to form additional preferred classes.

Physiologically acceptable arecolones and isoarecolones are the compounds described in the present document, including but not limited to those having the above R and $R^1$ substitutions, which are efficacious because they show agonist or antagonist activity, and which have properties such that they are safe for pharmaceutical use.

In another embodiment of this invention there is provided pharmaceutical formulations comprising as an active ingredient an effective amount of a compound of formula I or II and a pharmaceutically acceptable carrier, excipient or diluent therefor. Such formulations can be prepared for oral or parenteral administration for the treatment and prevention of Alzheimer's disease, Parkinson's disease, pain, diabetes, disorders of the gastrointestinal, central nervous and appetite regulatory systems of mammals, especially man.

For oral use of a compound of this invention, the selected compound can be administered, for example, in the form of tablets or capsules, or as an aqueous solution or suspension. In the case of tablets, common excipients include binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose and starch; fillers and carriers, for example, corn starch, gelatin, lactose, sucrose, microcrystalline cellulose, kaolin, mannitol, dicalcium phosphate, sodium chloride and alginic acid; lubricants such as magnesium stearate; disintegrants such as croscarmellose, microcrystalline cellulose, corn starch, sodium starch glycolate and alginic acid; and suitable wetting agents such as lauryl sulfate. For oral administration in capsule form, useful diluents include lactose and dried corn starch. When aqueous suspensions are desirable for oral use, the active ingredient can be combined with emulsifying and suspending agents, for example, sorbitol, methylcellulose, glucose/sugar syrup, gelatin, hydroxyethylcellulose, carboxymethylcellulose, aluminum stearate gel or hydrogenated edible oils, for example, almond oil, fractionated coconut oil, oily esters, propylene glycol or ethyl alcohol; flavoring agents such as peppermint, oil of wintergreen, cherry flavoring or the like; and preservatives such as methyl or propyl p-hydroxybenzoates or ascorbic acid.

The pharmaceutical formulations in accordance with this invention can also be prepared for parenteral use. Such formulations typically take the form of sterile isotonic solutions of the active ingredient according to standard pharmaceutical practice.

The appropriate dose of the compound of the present invention for its use as nicotinic receptor agonist or antagonist in humans will vary according to the age, weight and response of the individual patient, as well as the severity of the patient symptoms and the nature of the condition being treated. Thus, the preferred daily dose will normally be determined by the prescribing physician. However, in most instances, effective daily doses of the compounds of this invention will range from about 0.05 mg/kg/day to about 50 mg/kg/day and preferably about 1 mg/kg/day to about 25 mg/kg/day in a single or divided doses.

A further embodiment of this invention provides a process for preparing a compound of formula I or II, as shown below in Schemes I and II.

Scheme I, which describes a process for preparing a compound of formula I, used the ketone synthesis developed by Winreb and Nahm. Nahm, S. et al. Tetrahedron Letters, 22, 3815-3818 (1981). Oxalyl chloride, in the presence of a catalytic amount of dimethylformamide (DMF), converted a 1,2,5,6-tetrahydropyridine-3-carboxylic acid hydrobromide salt, 1-1, to its acid chloride. The acid chloride reacted with N,O-dimethylhydroxylamine in the presence of a tertiary amine base to give the corresponding amide, structure 1-2, in 80-90% yield. The amide could be purified by HPLC or isolated as its hydrochloride salt, but was normally used in subsequent reactions without further purification. In the presence of one to nine equivalents of an organometallic reagent at 0-60 °C, the amide 1-2 gave arecolones 1-3 in 5-80% yield with specific examples being given in

Table I. Reactive Grignard reagents gave good yields of arecolines, whereas less reactive Grignard reagents produced poorer results. The yields obtained with the less reactive Grignard reagents could be improved by conducting the reaction at ambient temperature or higher or by using the corresponding organometallic reagent.

Scheme I

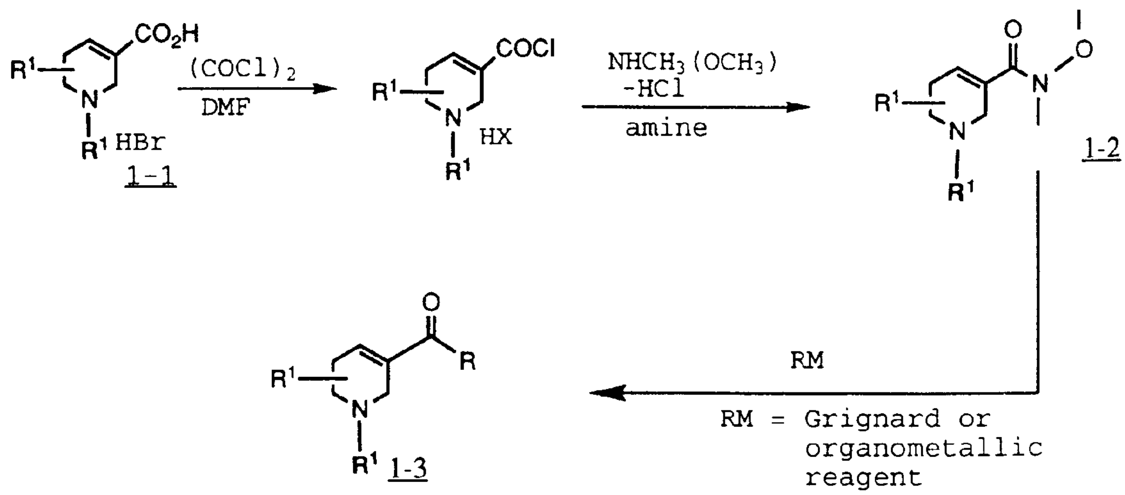


1-1 1-2 1-3

Table I

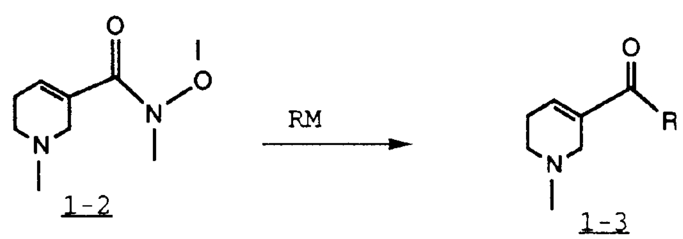


1-2 1-3

| R | M | Equivalents of RM | Temp °C | % Yield |
|---|---|---|---|---|
| $CH_3$ | MgCl | 2 | 0 | 79 |
| $CH_3CH_2$ | MgBr | 3 | 0 | 5 |
| $CH_3CH_2CH_2$ | MgCl | 2.2 | 0 | 11 |
| $CH_3CH_2CH_2CH_2$ | MgCl | 2 | 0 | 15 |
| $CH_3CH_2CH_2CH_2$ | MgCl | 1.5 | 22 | 35 |
| $CH_3CH_2CH_2CH_2$ | Li | 1.75 | 0 | 54 |
| $CH_3C{\equiv}C-$ | Li | 9 | 0 | 60 |

Scheme II describes the process for preparing a compound of formula II. Compound 2-1, 1,2,3,6-tetrahydropyridine-4-carboxylic acid hydrochloride, obtained from commercially available ethyl ester, was converted to its acid chloride with N,O-dimethylhydroxylamine to give the Nahm amide, structure 2-2, in 80-90% yield. The amide compound of structure 2-2, in the presence of one to nine equivalents of

Grignard reagent or organometailic reagent gave the isoarecolone compound 2-3, isolated in 65-75% yield as its hydrochloride salt.

Scheme II

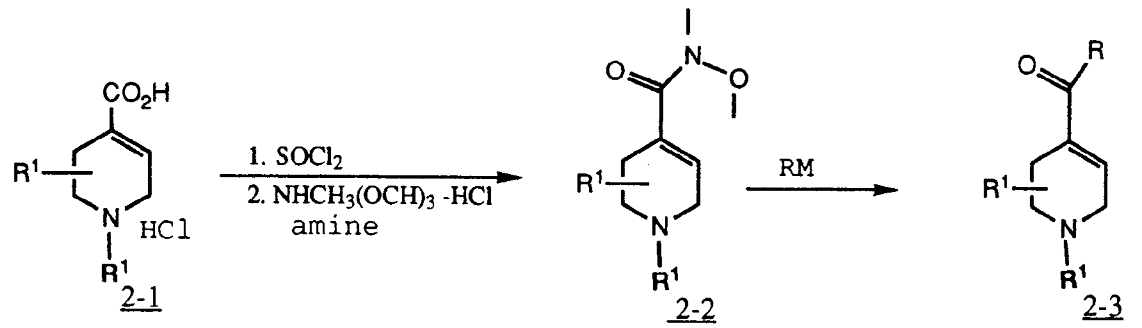


2-1 2-2 2-3

The following examples are provided to describe further the compounds of this invention and methods of preparation. They are thus provided for purposes of illustration only and are not to be construed as limiting the scope of the instant invention in any way.

Melting points were determined on a Mel-Temp apparatus and are uncorrected. A Waters PrepLC/500A using PrepPAK-500 silica gel cartridges, with the solvents specified, were used for hplc separations. Merck F254 silica gel plates were used for tlc. All reactions, exclusive of extraction procedures, were conducted under an argon atmosphere. A NMR Spectrometer, QE300, was employed for NMR measurements using the solvents described. No particular attempt was made to optimize reaction conditions for most of the reactions described.

**Preparation of N-Methoxy-1,N-dimethyl-1,2,5,6-tetrahydro-3-pyridinecarboxamide.**

Finely ground arecaidine hydrobromide, 47 g (0.21 mole), was suspended in a mixture of 1 liter of $CH_2Cl_2$ and 10 drops of dimethylformamide. To the mixture was added 50 g (0.39 mole) of oxalyl chloride and the reaction mixture was heated to a gentle reflux for 8 hours. After stirring overnight, the solvent was evaporated, another 500 ml of $CH_2Cl_2$ was added, and the solvent evaporated. The residue was suspended in 1 liter of $CH_2Cl_2$ and 23.25 g (0.27 mole) of N,O-dimethylhydroxylamine hydrochloride was added. The reaction mixture was cooled to 0 °C and 54 ml of pyridine was added dropwise with stirring. After the addition, the cooling was removed and the reaction stirred 4 hours. The solvent was evaporated and the residue dissolved in 100 ml of ice-water. The pH was adjusted to 10 with 5 N sodium hydroxide and the mixture extracted 3X with 150 ml of $CH_2Cl_2$ each time. The organic extracts were washed with brine, dried, and evaporated to give a yellow liquid. The liquid was treated with 200 ml of ether, the mixture filtered, and the solvent evaporated. After thorough drying in vacuo to remove residual pyridine, 34.3 g of N-methoxy-1,N-dimethyl-1,2,5,6-tetrahydro-3-pyridinecarboxamide was obtained (88% yield) of sufficient purity to be used in subsequent reactions. Analytically pure hydrochloride salt was obtained by treating the crude material with hydrochloric acid in ethyl acetate, mp 134-135 °C. pmr ppm (deuterium oxide) 2.7 (2H, m), 3.01 (3H, s), 3.35 (3H, s), 3.2-3.7 (2H, br m), 3.75 (3H, s), 3.8-4.2 (2H, br m), 6.65 (1H, m).

*Anal.* Calcd. for $C_9H_{16}N_2O_2$-HCl: C, 48.98; H, 7.76; N, 12.69. Found: C, 49.20; H, 7.95; N, 12.57.

Example 1

**1-(1,2,5,6-Tetrahydro-1-methyl-3-pyridinyl)-ethanone.**

A solution of 1.85 g (0.01 mole) of N-methoxy-1,N-dimethyl-1,2,5,6-tetrahydro-3-pyridinecarboxamide in 20 ml of dry tetrahydrofuran was cooled to 0 °C as 7 ml (0.021 mole) of 3 M methylmagnesium chloride in tetrahydrofuran was added dropwise. After 1.5 hours, the reaction mixture was poured into 30 ml ice-water containing enough 5 N hydrochloric acid to neutralize the amount of Grignard reagent used. The mixture was extracted 3X with 25 ml of $CH_2Cl_2$ and the combined organic extracts washed with brine. After drying and evaporating the solvent, 1.1 g of 1-(1,2,5,6-tetrahydro-1-methyl-3-pyridinyl)ethanone was obtained as a yellow liquid, 79% yield. The hydrochloride salt crystallized from 2-propanol, mp 213-214 °C. pmr ppm(deuterium oxide) 2.41 (3H, s), 2.79 (2H, m), 3.0 (3H, s), 3.13-3.3 (1H, m), 3.55-3.8 (2H, m), 4.1-4.25 (1H, m), 7.35 (1H, m).

*Anal.* Calcd. for $C_8H_{13}NO$-HCl: C, 54.70; H, 8.03; N, 7.77. Found: C, 54.78; H, 7.74; N, 7.92.

Example 2

**1-(1,2,5,6-Tetrahydro-1-methyl-3-pyridinyl)-1-propanone.**

A solution of 1.85 g (0.01 mole) of N-methoxy-1,N-dimethyl-1,2,5,6-tetrahydro-3-pyridinecarboxamide in 20 ml of dry tetrahydrofuran was cooled to 0 °C as 15 ml (0.03 mole) of 2 N ethylmagnesium bromide in tetrahydrofuran was added dropwise. After 1.5 hours, the reaction mixture was poured into 30 ml ice-water containing enough 5 N hydrochloric acid to neutralize the amount of Grignard reagent used. The mixture was extracted 3X with 25 ml of $CH_2Cl_2$ and the combined organic extracts washed with brine. After drying and evaporating the solvent, a yellow liquid was obtained that was purified by hplc eluting with an 8 liter gradient starting with $CH_2Cl_2$ and going to 7.5% methanol-1% ammonium hydroxide. The hydrochloride salt of 1-(1,2,5,6-tetrahydro-1-methyl-3-pyridinyl)-1-propanone crystallized from ethyl acetate as the dihydrate, 0.12 g, 5% yield, mp 112-114 °C. pmr ppm(deuterium oxide) 1.05 (3H, t), 2.75 (2H, m), 2.82 (2H, q), 3.0 (3H, s), 3.12-3.3 (1H, m), 3.5-3.85 (2H, m), 4.18 (1H, m), 7.35 (1H, m).

*Anal.* Calcd. for $C_9H_{15}NO$-$2H_2O$-HCl: C, 47.89; H, 8.93; N, 6.21. Found: C, 48.17; H, 8.65; N, 6.19.

Example 3

**1-(1,2,5,6-Tetrahydro-1-methyl-3-pyridinyl)-1-butanone.**

A solution of 1.85 g (0.01 mole) of N-methoxy-1,N-dimethyl-1,2,5,6-tetrahydro-3-pyridinecarboxamide in 20 ml of dry tetrahydrofuran was cooled to 0 °C as 8 ml (0.022 mole) of 2.8 M propylmagnesium chloride in ether was added dropwise. After 1.5 hours, the reaction mixture was poured into 30 ml ice-water containing enough 5 N hydrochloric acid to neutralize the amount of Grignard reagent used. The mixture was extracted 3X with 25 ml of $CH_2Cl_2$ and the combined organic extracts washed with brine. After drying and evaporating the solvent, a yellow liquid was obtained that was purified by hplc eluting with an 8 liter gradient starting with $CH_2Cl_2$ and going to 7.5% methanol-1% ammonium hydroxide. The liquid obtained was treated with dry hydrochloric acid in ether and the resulting solid recrystallized from ethyl acetate to give 0.22 g of 1-(1,2,5,6-tetrahydro-1-methyl-3-pyridinyl)-1-butanone hydrochloride, 11% yield, mp 106-108 °C. pmr ppm(deuterium oxide) 0.9 (3H, t), 1.62 (2H, m), 2.78 (4H, m), 3.0 (3H, s), 3.2-4.2 (4H, m), 7.35 (1H, m).

*Anal.* Calcd. for $C_{10}H_{17}NO$-HCl: C, 58.96; H, 8.91; N, 6.88. Found: C, 58.66; H, 8.93; N, 6.77.

Example 4

**1-(1,2,5,6-Tetrahydro-1-methyl-3-pyridinyl)-1-pentanone.**

A solution of 1.85 g (0.01 mole) of N-methoxy-1,N-dimethyl-1,2,5,6-tetrahydro-3-pyridinecarboxamide in 20 ml of dry tetrahydrofuran was cooled to 0 °C as 10 ml (0.02 mole) of 2 M *n*-butylmagnesium chloride in tetrahydrofuran was added dropwise. After 1.5 hours, the reaction mixture was poured into 30 ml ice-water containing enough 5 N hydrochloric acid to neutralize the amount of Grignard reagent used. The mixture was extracted 3X with 25 ml of $CH_2Cl_2$ and the combined organic extracts washed with brine. After drying and evaporating the solvent, a yellow liquid was obtained that was purified by hplc eluting with an 8 liter gradient starting with $CH_2Cl_2$ and going to 7.5% methanol-1% ammonium hydroxide. The 0.4 g of 1-(1,2,5,6-tetrahydro-1-methyl-3-pyridinyl)-1-pentanone obtained was converted to an oxalate salt that crystallized from ethanol as a white solid, 0.4 g, 15% yield, mp 175-176 °C. pmr ppm(deuterium oxide) 0.9 (3H, t), 1.32 (2H, m), 1.58 (2H, m), 2.75 (4H, m), 3.0 (3H, s), 3.25 (1H, m), 3.62 (1H, m), 3.7 (1H, d), 4.19 (1H, d), 7.35 (1H, m).

*Anal.* Calcd. for $C_{11}H_{19}NO$-$C_2H_2O_4$: C, 57.55; H, 7.80; N, 5.16. Found: C, 57.18; H, 7.75; N, 5.15.

**Preparation of 1-(1,2,5,6-tetrahydro-1-methyl-3-pyridinyl)-1-pentanone from butylmagnesium chloride at ambient temperature.**

A solution of 1.85 g (0.01 mole) of N-methoxy-1,N-dimethyl-1,2,5,6-tetrahydro-3-pyridinecarboxamide in 20 ml of dry tetrahydrofuran was stirred at ambient temperature during dropwise addition of 7.5 ml (0.015 mole) of 2 M n-butylmagnesium chloride in tetrahydrofuran. The reaction became warm during the addition. After the reaction had cooled to ambient temperature, the reaction was poured in 30 ml of ice-water containing 4 ml of 5 N hydrochloric acid. The mixture was extracted 3X with 25 ml of $CH_2Cl_2$, the extracts washed with brine, dried, and the solvent evaporated to give a yellow oil. Purification by hplc as in the previous example gave 0.75 g of yellow oil that was converted to 0.94 g of 1-(1,2,5,6-tetrahydro-1-methyl-3-pyridinyl)-1-pentanone

oxalate, 35% yield.

Example 5

**1-(1,2,5.6-Tetrahydro-1-methy)-3-pyridinyl)-2-butyn-1-one.**

A solution of 3.15 g (0.017 mole) of N-methoxy-1,N-dimethyl-1,2,5,6-tetrahydro-3-pyridinecarboxamide in 75 ml of dry tetrahydrofuran was cooled to 0 °C as 7 g (0.152 mole) of 1-lithiopropyne was added in 1 g portions in 5 minute intervals. After addition, the reaction mixture was stirred 20 minutes then carefully poured into 100 ml of an ice-brine mixture. The mixture was extracted 3X with 50 ml of $CH_2Cl_2$, the extracts washed with brine, dried, and the solvent evaporated to give 2.7 g of a yellow liquid. The liquid was converted to 2.6 g of oxalate salt, 60% yield, mp 147 °C dec. pmr ppm(deuterium oxide) 2.08 (3H, s), 2.8 (2H, m), 3.0 (3H, s), 3.17-3.27 (1H, m), 3.61 (1H, m), 3.75 (1H, d), 4.2 (1H, d), 7.61 (1H, m).

*Anal.* Calcd. for $C_{10}H_{13}NO\text{-}C_2H_2O_4$: C, 56.71; H, 5.97; N, 5.53. Found: C, 56.65; H, 5.80; N, 5.35.

**Preparation of N-Methoxy-N,1-dimethyl-1,2,3,6-tetrahydro-4-pyridinecarboxamide.**

A mixture of 26.5 g (0.149 mole) of 1-methyl-1,2,3,6-tetrahydro-4-pyridinecarboxylic acid and 100 ml of thionyl chloride was heated to reflux for 2 hours. The volatiles were evaporated, the residue treated with 200 ml of $CH_2Cl_2$ and the solvent evaporated. The resulting solid was suspended in 1.2 liters of $CH_2Cl_2$, 16.25 g (0.167 mole) of N,O-dimethylhydroxylamine hydrochloride was added and the mixture was cooled to 0 °C. Pyridine, 40.5 ml, was added dropwise, cooling was removed, and the reaction stirred 2 hours. The solvent was evaporated, the residue was suspended in 100 ml of ice-water, and the mixture made basic, pH 10, with 5 N sodium hydroxide. The mixture was extracted 3X with 100 ml of $CH_2Cl_2$, the extracts were dried, and the solvent was evaporated. The residue was suspended in ether, the mixture filtered, and the solvent evaporated to give 24.25 g of N-methoxy-N,1-dimethyl-1,2,3,6-tetrahydro-4-pyridinecarboxamide as a brown liquid, 88% yield. pmr ppm(deuteriochloroform) 2.4 (3H, s), 2.5 (2H, m), 2.58 (2H, t), 3.09 (2H, m), 3.2 (3H, s), 3.67 (3H, s), 6.25 (1H, m). This material was sufficiently pure to be used in subsequent reactions, but could be further purified by hplc eluting with an 8 liter gradient beginning with dichloromethane and going to 10% methanol. The hydrochloride salt crystallized from 2-propanol, mp 182-184 °C.

*Anal.* Calcd. for $C_9H_{16}N_2O_2\text{-}HCl$: C, 48.98; H, 7.76; N, 12.69. Found: C, 48.70; H, 7.67; N, 12.69.

Example 6

**1-(1,2,3,6-Tetrahydro-1-methyl-4-pyridinyl)-ethanone.**

A solution of 2.14 g (0.0116 mole) of N-methoxy-N,1-dimethyl-1,2,3,6-tetrahydro-4-pyridinecarboxamide in 20 ml of dry tetrahydrofuran was cooled to 0 °C as 8 ml (0.024 mole) of 3 M methylmagnesium chloride in tetrahydrofuran was added dropwise. After 1.5 hours, the reaction mixture was poured into 30 ml ice-water containing enough 5 N hydrochloric acid to neutralize the amount of Grignard reagent used. The mixture was extracted 3X with 25 ml of $CH_2Cl_2$ and the combined organic extracts washed with brine. After drying and evaporating the solvent a brown liquid was obtained that was treated with hydrochloric acid to provide 1.46 g of 1-(1,2,3,6-tetrahydro-1-methyl-4-pyridinyl)ethanone hydrochloride after recrystallization from 2-propanol, 71% yield, mp 166-167 °C. ppm(deuteriochloroform) 2.3 (3H, s), 2.38-2.5 (5H, m+s), 2.57 (2H, t), 3.17 (2H, m), 6.8 (1H, m).

*Anal.* Calcd. for $C_8H_{13}NO\text{-}HCl$: C, 54.70; H, 8.03; N, 7.97. Found: C, 54.57; H, 8.25; N, 7.62.

Example 7

**1-(1,2,3,6-Tetrahydro-1-methyl-4-pyridinyl)-propanone.**

To an ice cold solution of ethyllithium prepared from 1 g (0.14 mol) of lithium and 6.55 g (0.06 mol) bromoethane in 80 ml of pentane was added 1.85 g (0.01 mol) N-methoxy-N,1-dimethyl-1,2,3,6-tetrahydro-4-pyridinecarboxamide in 20 ml of ether. Cooling was removed and after 45 min the reaction was filtered through glass wool and poured into 30 ml ice-water. The organics were separated and the aqueous fraction extracted 2X with 50 ml of $CH_2Cl_2$. The extracts were dried and the solvent evaporated. The residue was dissolved in 25 ml of 2-propanol and treated with 1 g (0.011 mol) oxalic acid in 15 ml of 2-propanol. The mixture was heated on a steam bath and the volume adjusted to 60 ml with additional 2-propanol. Cooling produced a white precipitate that was collected and recrystallized from 2-propanol to give 1 g of 1-(1,2,3,6-tetrahydro-1-methyl-4-pyridinyl)propanone oxalate salt as tan flocculant crystals, mp 135.5 °C dec., 41 % yield. pmr ppm(deuterium oxide) 1.03 (3H, t), 2.5-2.76 (2H, m), 2.8 (2H, q), 2.96 (3H, s), 3.17-3.26 (1H, m), 3.59-3.64 (1H, m), 3.88 (1H, d), 4.1 (1H, d), 6.93 (1H, s).

*Anal.* Calcd. for $C_9H_{15}NO$-$C_2H_2O_4$: C, 54.31; H, 7.04; N, 5.76. Found: C, 54.28; H, 7.26; N, 5.70.

Example 8

**1-(1,2,3,6-Tetrahydro-1-methyl-4-pyridinyl)-butanone.**

To an ice cold solution of propyllithium prepared from 1 g (0.14 mol) of lithium and 7.45 g (0.06 mol) of bromopropane in 80 ml of pentane was added 1.85 g (0.01 mol) N-methoxy-N, 1-dimethyl-1,2,3,6-tetrahydro-4-pyridinecarboxamide in 20 ml of ether. Cooling was removed and after 45 min the reaction was filtered through glass wool and poured into 30 ml of ice-water. The organics were separated and the aqueous fraction extracted 2X with 50 ml of $CH_2Cl_2$. The extracts were dried and the solvent evaporated. The residue was dissolved in 25 ml of 2-propanol and treated with 1 g (0.011 mol) of oxalic acid in 15 ml of 2-propanol. The mixture was heated on a steam bath and the volume adjusted to 60 ml with additional 2-propanol. Cooling produced 2.08 g of 1-(1,2,3,6-tetrahydro-1-methyl-4-pyridinyl)butanone oxalate salt as flocculant crystals, 81 % yield. Recrystallization from 2-propanol gives analytically pure material, mp 128 °C dec. pmr ppm(deuterium oxide) 0.87 (3H, t), 1.58 (2H, m) 2.55-2.65 (2H, m), 2.75 (2H, t), 2.97 (3H, s), 3.16-3.26 (1H, m), 3.6-3.7 (1H, m), 3.86 (1H, d), 4.16 (1H, d), 6.95 (1H, s).

*Anal.* Calcd. for $C_{10}H_{17}NO$-$C_2H_2O_4$: C, 56.02; H, 7.44; N, 5.44. Found: C, 55.80; H, 7.71; N, 5.34.

Example 9

**1-(1,2,3,6-Tetrahydro-1-methyl-4-pyridinyl)-pentanone.**

A solution of 1.85 g (0.01 mole) of N-methoxy-1,N-dimethyl-1,2,3,6-tetrahydro-4-pyridinecarboxamide in 20 ml of tetrahydrofuran was cooled to 0 °C as 8 ml (0.0128 mol) of 1.6 M *n*-butyllithium in hexane was added dropwise. After 15 min, the excess butyllithium was destroyed with 1 ml of 2-propanol followed by 10 ml of 1 N hydrochloric acid in ice. The organics were separated and the aqueous phase extracted 2X with 50 ml of $CH_2Cl_2$. The extracts were dried and the solvent evaporated to give a brown liquid that was converted to 1.29 g of 1-(1,2,3,6-tetrahydro-1-methyl-4-pyridinyl)-1-pentanone hydrochloride after recrystallization from 2-propanol, mp 150-151 °C, 59 % yield. pmr ppm(deuterium oxide) 0.87 (3H, t), 1.3 (2H, m), 1.58 (2H, m), 2.65 (2H, m), 2.8 (2H, t), 2.9 (3H, s), 3.3 (1H, m), 3.62 (1H, m), 3.9 (1H, m), 4.12 (1H, m), 6.9 (1H, m).

*Anal.* Calcd. for $C_{11}H_{19}NO$-HCl: C, 60.68; H, 9.26; N, 6.43. Found: C, 60.43; H, 9.23; N, 6.60.

Example 10

**1-(1,2,3,6-Tetrahydro-1-methyl-4-pyridinyl)]-2-butyn-1-one.**

To a solution of 1.85 g (0.01 mol) of N-methoxy-1,N-dimethyl-1,2,3,6-tetrahydro-4-pyridinecarboxamide in 20 ml of dry tetrahydrofuran was added in portions 4 g (0.087 mol) of 1-lithiopropyne. After addition, the reaction was stirred 1 hr then carefully poured into a mixture of ice and 10 ml of 5N HCl. The mixture was extracted 3X with 50 ml of $CH_2Cl_2$, the extracts washed with brine, dried, and the solvent evaporated. The residue was converted to an oxalate salt and recrystallized from ethanol to give 0.59 g of 1-(1,2,3,6-tetrahydro-1-methyl-4-pyridinyl)-2-butyn-1-one oxalate, mp 118 °C dec., 23 % yield. pmr ppm(deuterium oxide) 2.1 (3H, s), 2.5-2.75 (2H, m), 3.4 (1H, m), 3.65 (1H, m), 3.92 (1H, d), 4.2 (1H, m), 7.3 (1H, s).

*Anal.* Calcd. for $C_{10}H_{13}NO$-$C_2H_2O_4$: C, 56.71; H, 5.97; N, 5.53. Found: C, 56.49; H, 6.05; N, 5.39.

Example 11

**1-(1,2,3.6-Tetrahydro-1-methyl-4-pyridinyl)-2-methylbutanone.**

A solution of 1.85 g (0.01 mole) of N-methoxy-1,N-dimethyl-1,2,3,6-tetrahydro-4-pyridinecarboxamide in 20 ml of tetrahydrofuran was cooled to 0 °C as 15 ml (0.0195 mol) of 1.3 M *sec*-butyllithium in cyclohexane was added dropwise. After 1 hr, the reaction was poured into 50 ml of ice-water. The organics were separated and the aqueous phase extracted 2X with 50 ml of $CH_2Cl_2$. The extracts were dried and the solvent evaporated.

The residue was converted to an oxalate salt and recrystallized from 2-propanol to give 0.9 g of 1-(1,2,3,6-tetrahydro-1-methyl-4-pyridinyl)-2-methylbutanone oxalate, mp 149-151 °C dec., 33 % yield. pmr ppm(deuterium oxide) 0.82 (3H, t), 1.05 (3H, d), 1.45 (1H, m), 1.62 (1H, m) 2.45-2.8 (2H, m), 3.0 (3H, s), 3.19-3.37 (2H, m), 3.6-3.7 (1H, m), 3.9 (1H, d), 4.15 (1H, d), 7.02 (1H, s).

*Anal.* Calcd. for $C_{11}H_{19}NO-C_2H_2O_4$: C, 57.55; H, 7.80; N, 5.16. Found: C, 57.25; H, 7.88; N, 5.46.

Example 12

**1-(1,2,3,6-Tetrahydro-1-methyl-4-pyridinyl)-2,2-dimethylpropanone.**

A solution of 1.85 g (0.01 mole) of N-methoxy-1,N-dimethyl-1,2,3,6-tetrahydro-4-pyridinecarboxamide in 75 ml of ether was cooled to 0 °C as 10 ml (0.015 mol) of 1.5 M t-butyllithium in pentane was added dropwise. After 30 min, the reaction was poured into 50 ml of ice-water. The organics were separated and the aqueous phase extracted 2X with 50 ml of $CH_2Cl_2$. The extracts were dried and the solvent evaporated. The residue was converted to an oxalate salt and recrystallized from ethylacetate to give 0.91 g of 1-(1,2,3,6-tetrahydro-1-methyl-4-pyridinyl)-2,2-dimethylpropanone oxalate, mp 109-110 °C, 34 % yield. pmr ppm(deuterium oxide) 1.23 (9H, s), 2.65 (2H, m), 2.95 (3H, s), 3.19-3.28 (1H, m), 3.57-3.63 (1H, m), 3.8 (1H, d), 4.06 (1H, d), 6.5 (1H, s).

*Anal* Calcd. for $C_{11}H_{19}NO-C_2H_2O_4$: C, 57.55; H, 7.80; N, 5.16. Found: C, 57.26; H, 7.73; N, 5.37.

Binding to rat brain nicotinic receptors was performed using the method of Abood and Grassi. Abood, L.E. et al., Biochem. Pharmacol. 35:4199-4202 (1987). Frozen rat brain cortices (Pel-Freez Biologicals, Roger, AR) were homogenized using a Polytron (Binkmann PT-10; setting 6 for 15 sec) in 30 volumes of buffer (50 mM tris, 120 mM sodium chloride, 5 mM potassium chloride, 2 mM calcium chloride, 1 mM magnesium chloride, pH 7.3 at 4°C). The homogenate was centrifuged at 1,000 xg for 10 min. (Sorval RC-5B; SA600 rotor). The supernatant was then centrifuged at 40,000 xg for 20 min. The final pellet was resuspended in buffer at a concentration of 12 vol./gram wet weight of tissue. Final protein concentrations for each assay were determined using the Lowry et al. method. Lowry, O.H. et al., J. Biol. Chem. 193:265-275 (1951).

[$^3$H]N-Methyl-carbamyl choline iodide ([$^3$H]MCC) binding to rat cortices was measured by filtration. Compounds dissolved in distilled water at concentrations of 1 nM to 1 mM, were incubated with [$^3$H]MCC (1 nM), membrane aliquots (approximately 1.5 mg./ml. protein) and buffer, in a final volume of 0.5 ml. Triplicate samples were incubated at 4°C for 1 hour. Nonspecific [$^3$H]MCC binding was determined in the presence of 10 mM nicotine. All [$^3$H]MCC assays were terminated by filtering the samples over Whatman GF/B glass-fiber filters on a Brandel cell harvester, followed by a 10 ml. ice cold saline wash. Filters were pre-soaked in 0.05% polyethyleneimine.

Radioactivity bound was determined after a period of equilibration (at least 5 hr) in Beckman Ready Protein Scintillation Cocktail using a Beckman LS5000 TA counter with an efficiency of 43%.

All assays were performed in triplicate, and the mean values of three separate experiments were used to obtain the affinity constants. Analysis of the data was performed using nonlinear least-squares regression. Munson, P.J. and Rodbard, J.D., Anal. Biochem. 107:220-239 (1980).

Materials

[$^3$H]N-methyl-carbamyl choline iodide, specific activity 84.0 Ci/MMol) was purchased from New England Nuclear (Boston, MA). All other reagents were purchased from Sigma Chemical Company (St. Louis, MO).

The hippocampus from male Sprague-Dawley rats was homogenized in 10 volumes of 0.32 M sucrose, centrifuged at 1000 x g for 10 min, and the supernatant was centrifuged at 17,000 x g for 20 min. The synaptosoma! fraction ($P_2$) pellet was homogenized in 50 volumes of 20 mM Tris-Cl buffer, pH 7.4, and centrifuged at 50,000 x g for 10 min. After resuspension in buffer, the suspension was preincubated for 30 min at 4 °C, and centrifuged again. The pellet was resuspended in 3 volumes of buffer and frozen at -70 °C until used.

The inhibition of binding of oxotremorine-M to hippocampal membranes was determined by adding unlabeled drug, 3 nM $^3$H-oxotremorine-M (87 Ci/mmol, New England Nuclear), and hippocampal membranes equivalent to 10 mg tissue wet weight (about 0.1 mg protein) in 1 mL total volume of 20 mM Tris-C1 buffer, pH 7.4. For oxotremorine-M binding, the homogenates were incubated at 25 °C for 15 min. After incubation, the homogenates were filtered through Whatman GF/C filters with vacuum. The filters were rinsed 3X with 1 mL of cold buffer and placed in scintillation vials containing Ready Protein+ (Beckman) scintillation fluid. Radioactivity trapped on the filters was determined by liquid scintillation spectrometry. Non specific binding was determined using 1 μM atropine.

The concentration of compound required to inhibit binding 50% ($IC_{50}$) was calculated using the ALLFIT program.

Tables II and III show the affinity of these compounds for nicotinic and muscarinic receptors as determined by [$^3$H]N-methyl-carbamyl choline iodide([$^3$H]MCC) and [$^3$H]oxotremorine ([$^3$H]OXO) bindings, respectively, to rat cortical membranes. These compounds, however, show selective affinity for nicotinic receptors as compared to muscarinic receptors, i.e. OXO/MMC >1.

Table II

Nicotinic Receptor Affinity of Arecolones

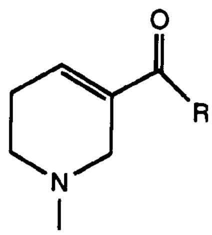

| R | OXO | MCC | $K_i$ Ratio OXO/MCC |
|---|---|---|---|
| $CH_3$ | 4380 | 32.7 | 134 |
| $CH_2CH_3$ | 2818 | 5.8 | 486 |
| $(CH_2)_2CH_3$ | 2730 | 13.1 | 208 |
| $(CH_2)_3CH_3$ | 1385 | 61.3 | 23 |
| $CH_3C{\equiv}C-$ | 1650 | 5.8 | 284 |

Table III

Nicotinic Receptor Affinity of Isoarecolones

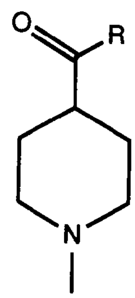

| R | OXO | MCC | $K_i$ Ratio OXO/MCC |
|---|---|---|---|
| $CH_3$ | 5425 | 48.4 | 112 |
| $CH_2CH_3$ | >5000 | 25.7 | >195 |
| $(CH_2)_2CH_3$ | >5000 | 67.3 | >74 |
| $(CH_2)_3CH_3$ | 4345 | 148.7 | 29 |
| $CH_3C{\equiv}C-$ | 2690 | 22.0 | 122 |
| 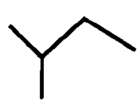 | 4785 | 224 | 21 |
| 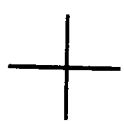 | 6250 | 56.7 | 110 |

## Claims

1. A compound of the formula

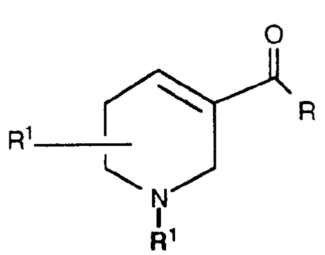

I

or

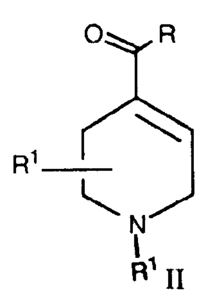

II

wherein

R is $C_2$-$C_6$ alkyl, $C_2$-$C_6$ alkenyl, $C_2$-$C_6$ alkynyl, $C_2$-$C_6$ alkoxyalkyl, $C_2$-$C_6$ alkylthioalkyl, $C_2$-$C_6$ alkylaminoalkyl, $C_3$-$C_6$ cycloalkyl, $C_1$-$C_6$ alkyl substituted with $C_3$-$C_6$ cycloalkyl, or $C_1$-$C_6$ polyfluoroalkyl;

each $R^1$ independently is hydrogen or $(C_1$-$C_3)$ alkyl; or a physiologically-acceptable acid-addition salt thereof for use as a pharmaceutical.

2. A compound of Claim 1 of the formula

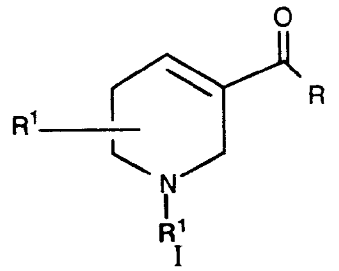

I

for use as a pharmaceutical.

3. A compound of Claim 1 of the formula

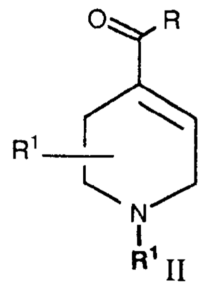

II

for use as a pharmaceutical.

4. A compound of Claim 1 wherein R is $C_2$-$C_6$ alkyl.

5. A compound of Claim 4 wherein R is $-CH_2CH_3$.

6. An arecolone or isoarecolone as defined in any one of Claims 1 to 5 for use in treating a mammal with a central nervous system related disorder wherein said disorder is related to nicotinic agonist or antagonist activity.

7. A pharmaceutical formulation comprising as an active ingredient an arecolone or isoarecolone as defined in any one of Claims 1 to 5, or a physiologically-acceptable acid addition salt thereof, associated with one or more pharmaceutically acceptable carriers, excipients, or diluents therefor.

8. A process for preparing an arecolone as defined in any one of Claims 1 to 5, which comprises reacting an appropriately substituted 1,2,5,6-tetrahydropyridine-3-carboxylic acid with N,O-dimethylhydroxylamine in the presence of a Grignard or organometallic reagent.

9. A process for preparing an isoarceolone as claimed in any one of Claims 1 to 5, which comprises reacting an appropriately substituted 1,2,3,6-tetrahydropyridine-4-carboxylic acid with N,O-dimethylhydroxylamine in the presence of a Grignard or organometallic reagent.

European Patent Office

# EUROPEAN SEARCH REPORT

Application Number

EP 92 30 9339

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-4 710 508 (WARNER-LAMBERT COMPANY) 1 December 1987<br>*see formula 9, column 8 and examples 4,13*<br>--- | 1-5 | C07D211/70<br>A61K31/445 |
| X | J. HETEROCYCLIC CHEM.<br>vol. 27, 1990,<br>pages 1709 - 1712;<br>J.S.WARD AND LEANDER MERRITT: 'The improved convergent synthesis of Arecolones and Isoarecolone'<br>* see Table I, page 1710*<br>--- | 1,2,4,5,8 | |
| Y | JOURNAL OF MEDICINAL CHEMISTRY<br>vol. 31, 1988,<br>pages 545 - 554;<br>J.A.WATERS ET AL: 'Synthesis, Pharmacology and Molecular modeling studies of semirigid nicotinic agonists'<br>--- | 1-7 | |
| Y | EP-A-0 288 394 (ROUSSEL-UCLAF) 26 October 1988<br>--- | 1-7 | TECHNICAL FIELDS SEARCHED (Int. Cl.5 ) |
| O,Y | MOLECULAR PHARMACOLOGY<br>vol. 36, 1989,<br>pages 177 - 184;<br>C.E.SPIVAK ET AL: 'Binding of semirigid nicotinic agonists to nicotinic and muscarinic receptors'<br>----- | 1-7 | C07D<br>A61K |

The present search report has been drawn up for all claims

| Place of search | Date of completion of the search | Examiner |
|---|---|---|
| MUNICH | 20 NOVEMBER 1992 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)